# EUROPEAN PATENT APPLICATION

(11) **EP 2 163 319 A2**
(43) Date of publication of application: **17.03.2010**
(21) Application number: 09169969.4
(22) Date of filing: 10.09.2009
(51) Int. Cl.: B09B 3/00, B01J 3/04, A61L 11/00, B03B 9/06

(54) **Process for waste treatment**

(30) Priority: 11.09.2008 GB 0816618
(71) Applicant: Sterecycle Ltd., London W8 5SF (GB)
(72) Inventor: Campion, Philip, Stockport, SK8 8AT (GB); Greenwell, Paul, London, W8 5SF (GB)
(74) Representative: den Hartog, Jeroen H.J.

(57) **Abstract**

The present invention relates to a process to treat municipal solid waste comprising the steps of, feeding waste material into a pressure vessel (6,6'), closing the vessel and optionally evacuating the contents of air, agitating the vessel while having steam pressure in the vessel at about 2.5 bar (abs) or higher, and about 4.8 bar (abs) or lower, and a temperature of about 127 °C or higher and about 148 °C or lower for at least about 15 min, reducing the pressure in the vessel, optionally evacuating the contents of the vessel to 0.6 bar abs or lower, discharging the vessel, classifying the treated waste, wherein steam at about 165 °C or more and a pressure of about 5 bar (abs) or more, is injected into the vessel after closing to raise the pressure within the vessel to 2.5-4.8 bar (abs) in a condensing atmosphere, and, for a suitable period preferably continue to inject steam at a reduced flowrate to allow heating of the waste material by steam condensation, and thereby maintain the waste treatment conditions. The improvement allows more efficient, faster process, while using less energy and water, reducing the effluent burden.

## Description

The invention relates to a process for waste treatment.

Waste, in particular municipal solid waste (MSW) and similar types of commercial waste, is an increasing burden on landfills and other forms of waste treatment. Part of the waste stream may be recycled through separated collection. Part of the landfill may be circumvented by incineration; however this is very costly and may have negative environmental impacts. One of the alternatives suggested is treating the waste in an autoclave with steam while tumbling the content in order to breakdown the organic content and enable easier separation. Several examples of this idea exists, such as disclosed in EP 0771237 , US 4844351, US 6306248, US 4974781, WO 03/09299, WO 2004/018767 and WO 03/025101.

As of yet, none of these technologies have been commercially put into practice in treating MSW, since several practical problems exist. For example, treated waste can be separated into for example a fibre fraction which is useful as a renewable fuel and may be eligible for enhanced payments for power generation e.g. by the issue of Renewable Obligation Certificates or their equivalent, but in practice it is difficult to obtain fibre with the necessary low levels of contamination and in high yield. Plastics, glass and grit are still present in the fibre, which can very adversely affect economic use in combustion systems. Glass and grit increase the quantity of bottom ash and may also adversely affect its handling characteristics. In case there is too much plastic, the fibre is not entitled to ROC status, and/or requires extensive emissions control measures to meet Environmental Emission limits set by the EC Waste Incineration Directive or other equivalent legislation, or the fibre separation from the treated waste is such that the recovery yield of the clean fibre is much less than e.g. 90%. Further, apparatus suggested in the prior art may not be economical nor efficient, since the vessels for commercial operation will be about 20 cubic metres or more, generally even 30 cubic metres or more likely up to 60 cubic metres, and even 120 cubic metres. For such large vessels energy consumption is high and not sufficiently attractive for a commercially acceptable process. Furthermore, the contaminated gases emitted and fluids discharged as effluents are also substantial and not readily acceptable in commercial use, or the treatment thereof requires substantial investments. And, last but not least, the cycle time needs to be sufficiently 'short', in order to treat as much waste as possible. So, the current processes are still to be improved so that these disadvantages are eliminated.

The technology however, has interesting potential: The organic fibres could be used as clean biomass fuel or compost; the glass/grit fraction - if sufficiently clean - can be used either by separating the glass for uses in recycled glass products, or it can be used as recycled aggregates. The municipal waste does not have to be pre-selected, and is reduced to about 30 volume% of its original size. Further, when fibres are separated, and other useful materials are recycled, landfill can be limited to less than 10 volume% of the original waste.

In view of global warming, there is a further need to provide so-called 'green'-electricity, being electricity obtained from fully renewable resources.

It is an object of the present inventions to solve one or more of the above problems, and to improve the prior art processes in one or more aspects. It is a further object of the invention to provide a method for obtaining electricity from renewable resources in an efficient and effective way.

For the first embodiment of the invention, the object is to provide a more effective process to treat MSW with improved cycle times.

In one embodiment of the invention, the process to treat municipal solid waste comprises the steps of,
(1) feeding waste material into a pressure vessel
(2) closing the vessel and optionally evacuating the contents of air
(3) agitating the vessel while having steam pressure in the vessel at about 2.5 bar abs or higher, and at about 4.8 bar abs or lower, and a temperature of about 127 °C or higher and of about 148 °C or lower for at least about 15 min
(4) reducing the pressure in the vessel
(5) optionally evacuating the contents of the vessel to 0.6 bar (abs) or lower
(6) discharging the vessel
(7) classifying the treated waste
wherein after step (2) steam at a temperature of about 165 °C or more and a pressure of about 5 bar (abs) or more, is injected into the vessel after closing to raise the pressure within the vessel to 2.5-4.8 bar (abs) in condensing atmosphere, and thereafter in step (3) preferably, for a suitable period continue to inject steam at a reduced flow rate to allow heating of the waste material by steam condensation, and thereby maintain the waste treatment conditions.

Preferably, the temperature in step 3 is about 148 °C or lower, preferably, the temperature is about 130 °C or higher. The pressure is preferably about 4.7 bar (abs) or lower in vessel in steps (3), and preferably about 3 bar (abs) or higher.

Preferably dry, saturated steam of a temperature of about 170 °C or more is injected into the vessel, even more preferably of about 184 °C or more. For practical reasons, a temperature of about 240 °C or less is used, preferably a temperature of about 224 °C or less. The pressure of the steam may be about 8 bar (abs) or higher, or about 11 bar (abs) or higher. In practice, a pressure of about 25 bar (abs) or less is used, but if higher pressure steam would be available on a site, like for example 50 bar (abs) steam, this could be used as well.

Preferably, the time of high pressure steam injection to reach the vessel operating pressure of about 3-4.8 bar (abs) is about 30 min or less, but can be about 20 min or less, depending on the capacity of the steam boiler, accumulator, and cross section area of the pipes. Generally, the time will be 2 min or more, and likely about 5 min or more. Obviously, a shorter time has an advantage of shorter cycle times.

Preferably, after closing of the vessel, and before injecting the steam, a vacuum is applied, while using a vacuum system, and a condenser or scrubber or combined condenser/scrubber is used to treat the extracted waste gas steam. The vacuum can be applied while using an eductor, an ejector set or - preferably - a vacuum pump. The removal of air from the vessel ensures that the steam condensation conditions within the vessel during heat up and during the waste treatment are maintained under optimum pressure and temperature which match those of saturated steam, thereby the adverse effects of air within the vessel on both condensation and operating temperature are eliminated

Also preferably after treatment of the waste and during or after reducing the vessel pressure towards atmospheric pressure i.e. below 2.5 bar (abs), preferably below 2 bar (abs) and most likely below 1.5 bar (abs) a vacuum is applied to the vessel using a vacuum system, and a condenser or scrubber or combined condenser/scrubber to treat the extracted waste gas steam. The vacuum can be applied while using an eductor, an ejector set or - preferably - a vacuum pump.

A vacuum system is defined in this invention as a mechanically driven pump, eductor, ejector set or other device that is designed to displace a gas or vapour and in so doing reduces the pressure in any vessel or equipment to which it is connected to below atmospheric pressure.

It is preferred to achieve a vacuum of about 0.6 bar (abs) or lower, as better drying of the fibres is achieved, including further expansion and break down. Generally, the vacuum will be about 0.1 bar (abs) or higher, as a lower vacuum will take longer to achieve, and may be less economical. Preferably, the vacuum applied in step (3) is about 0.5 bar (abs) or lower, and most preferably about 0.4 bar (abs) or lower. Generally, the vacuum can be about 0.2 bar (abs) or higher, and may be about 0.3 bar (abs) or higher.

Another advantage of using a vacuum system is that in depressurizing the vessel, time is gained (each batch will have a shorter cycle time in comparison to simply letting pressure off to atmospheric), and even more importantly, that when opening the vessel after the autoclave treatment, no live steam is emitted and vapours displaced during emptying the autoclave contain only extremely small concentrations of Volatile Organic Compounds. This can be further improved, by letting in fresh air, while still having the vacuum pump working. By cooling the contents by evacuation and air purging, even less steam will be emitted. Letting in fresh air preferably is done after drying at reduced pressure has been effected for some time.

The vapours extracted can be condensed in a condenser, and recirculated in the system. Preferably, cleaning of the gas e.g. air purge is achieved in a scrubber.

A scrubber may be defined as an apparatus in which a contaminated gas flow is directly contacted with a liquid flow, within which the VOC components and other contaminants have solubility and where the liquid stream is at a lower temperature than the gas. Thus the liquid stream is able to dissolve VOCs, condense steam, and capture other contaminants present in the gas stream. The scrubber can also be designed to capture a high proportion of the total contaminants in the gas stream via the use of a range of multi-stage contact internal designs

Further optimisation with respect to environmental burden is achieved when the scrubbing liquid is recirculated. The gas may comprise steam, volatile organic compounds (VOC's) and dust. In the scrubber, steam is condensed, increasing the extraction rate of steam and/or air from the vessel.

Hence, in a preferred embodiment of the invention, the fluid to clean the exhaust gas is water. More preferably, the water is process water that is recycled in the system. This is an advantage, as much of the dust and volatile components may be kept in the waste treating cycles, and may be broken down in a next cycle. The process water can be used to wet the MSW material, and to produce steam within the vessel. There is no need to use boiler quality feed water for these purposes.

The scrubber of the present invention, depending on autoclave size, only needs a pump capacity of say 200 cubic metres or less per hour, and at a pressure of for example 2 bar gauge to be effective. The scrubber may be, for example, a spray condenser.

Preferably, two or more vessels are used in an alternating fashion, while using the heat and steam from the discharge of step (4) to apply heat and/or steam to another vessel in a step (3'). This is also an advantage for efficient use of the vacuum system, as one system comprising for example one pump or set of pumps can be used intermittently for the different vessels. Hence, only one system is needed for processing with two or more vessels. It is particularly preferred to have two or more vacuum pumps in the system as in case the one needs maintenance, it is still possible to operate the plant.

In a preferred embodiment of the invention, the process comprises further a step of applying a vacuum after charging of the vessel, but before adding steam thereto. This has as advantage that pressure is more effectively increased to obtain a fully saturated environment. It furthermore has as advantage that the air displaced by the vacuum system - which does include some VOC's - is effectively cleaned in the scrubber or condenser. Prior art suggests to add steam during loading to displace air. This has as a disadvantage that a large contaminated air/steam stream is emitted to the environment, or has to be handled, and that this requires increased energy because of the steam.

In one further preferred embodiment of the invention, the process to treat municipal solid waste in two or more vessels, which are used in an alternating fashion, additionally comprises the steps of, depressurizing the vessel, and transferring the steam from a first vessel to a second vessel,

In a further preferred embodiment, the process additionally comprises the step of, further transferring steam from the first vessel with the aid of a compressor while using high pressure steam, which is also introduced in the pressure vessel. A process with using an apparatus for increasing the speed of steam transfer from one vessel to another has advantages. Firstly, the time to discharge one, and to charge another vessel is shortened, secondly, more efficient use is made of steam, as energy losses are diminished, and thirdly, more steam can be extracted from the first vessel, and less steam is necessary for fresh steam supply.

The compressor preferably is a steam powered compressor. As steam powered or driven compressors, it is suitable to use a steam ejector, steam eductor or steam driven jet pump. In the present application, these variants are together denoted as thermo-compressor.

The compressor is preferably a thermo-compressor, driven by steam of e.g. a pressure between 6 and 20 or 6 and 15 bar gauge, which steam is supplied to the vessel. This is in particular an advantage as fresh high pressure steam is used to charge steam to the vessels.

Adding fresh steam at the high temperature has not only the advantage of shortening the cycle-time. With shortening the cycle-time, the capacity of a plant is increased. It appeared furthermore, that in total less water is needed, and that fibres are more efficiently broken up. Yet, the superheated steam is cooled down inside the vessel to below the condensation temperature, whereby the condensing steam precludes the vessel from becoming contaminated with molten plastic.

The fresh high temperature steam can be released from an accumulator, and/or from a steam boiler. The boiler or accumulator preferably is able to release about 3 tonnes or more, preferably, about 5 tonnes or more of steam at 15 bar gauge in 10 to 15 min, preferably about 8 tonnes of steam or more. Generally, the supply will be 25 tonnes or less, preferably about 15 tonnes or less per 10 to 15 min because of economic reasons. A suitable amount may be for example 10 ton of steam at 15 bar gauge. In case other pressures are available, it may be equally suitable to use another pressure, like 8 bar gauge or higher, and like 40 bar gauge or lower, preferably 25 bar gauge or lower.

The steam at the cited pressure will have a certain saturation temperature, as can be found in a steam table. Preferably, steam with a pressure of 3 bar higher pressure than the operating pressure of the autoclave is used for addition of steam to the autoclave, even more preferably about 4 bar pressure difference, and even more preferably even about 5 bar pressure difference.

Generally, an autoclave has a pressure safety valve to preclude a too high pressure inside the autoclave. It may be suitable to have a pressure reduction valve on the steam inlet of the autoclave. In such case, the pressure is for example lowered from 10 to 5 bar (abs) just before the steam enters the autoclave. This has virtually the same effect as letting steam in at 10 bar, as the steam would expand immediately in the vessel to the pressure which is present in the vessel. In both cases, superheated steam is effectively used to heat the content of the vessel. It is preferred, that the steam entering the vessel is superheated for about 30 °C or more. Preferably the steam entering the vessel is superheated for about 50 °C or more. Generally, the steam is superheated for about 100 °C or less

The superheated steam causes quick heating of the content of the vessel, with relatively small amount of water. Fibres comprising water will rapidly heat by contact with the incoming superheated steam which will aid breakdown of the heated material and aid formation of fibre. As the steam condenses further rapid heat transfer occurs together with wetting of the vessel, plastic and other components. In this way fouling of the vessel is diminished.

In a further preferred embodiment of the invention, the process to treat municipal solid waste in two or more vessels, which are used in an alternating fashion, additionally comprises the step of heating the content of the vessel through indirect heating with thermal fluid. The indirect heating may take place from loading, or from cooking, or in the drying stage.

Preferably, the indirect heating is at least applied during the drying phase, as dryer fibrous materials can be obtained in this way.

Preferably, the thermal fluid is provided via a heating system which comprises a primary circuit with a heating element for heating the thermal fluid, and secondary circuits (one circuit for each vessel) for heating the two or more vessels independently.

Preferably, the secondary circuits comprise a cooling element to lower the temperature of the thermal fluid. This may be important, as the inner surface of the vessel and the coils in the vessel preferably are at a temperature of about 100 °C or lower during unloading, and preferably during the time the vessel is at atmospheric pressure or lower, as otherwise plastic may stick to the walls of the vessel. A suitable cooling device may comprise a fin-fan cooler in the secondary circuit. Another cooling device may consist of a liquid/liquid-heat exchanger. Alternatively, one could exchange the hot for cold oil, supply the hot oil to the primary circuit and have a supply tank for cold oil to draw the cooling fluid from. If a supply tank for cold oil is used, it is preferred to have a cooling element in the cold oil tank, as that constitutes an effective means of cooling.

In addition, or otherwise, sticking of plastic may be limited or prevented by having the surface of the internals wet. This may be disadvantageous during unloading if the content preferably is relatively dry. Wetting can be more useful during loading. It is an unexpected advantage of the present invention, that the condensing saturated steam causes sufficient wetness on the surface of the vessel, that melting and sticking of plastic is circumvented.

In another preferred embodiment of the process, indirect heating with the thermal fluid is applied as soon as the vessel is charged with waste, and the door is closed.

The thermal fluid can be heated to a temperature of about 110 °C or higher, preferably of about 120 °C or higher, such as for example up to about 130 °C. Higher temperatures are feasible, but may become expensive as special measures often have to be taken. Generally, the temperature of the thermal fluid is about 300 °C or lower. In view of the potential decomposition of plastics, it is preferred to keep the temperature of the thermal fluid at about 200 °C or lower.

As thermal fluid, mineral oils or synthetic fluids are suitable. Synthetic fluids are preferred due to their lower flashpoint and higher auto-ignition temperatures.

The heating is achieved by heating thermal fluid in the first circuit by for example direct fired heating coils containing the thermal fluid, or by electrical heating or the like.

In another embodiment of the invention, the indirect heating of the autoclave vessel is done at least in part through a heated jacket. All the indirect heating can be supplied through the jacket. In a preferred embodiment, heating is done through both conduits in the vessel, and a heated jacket which shortens the cycle time.

In a further preferred embodiment, the process allows independent closure of the heating lines to either the jacket or the internal conduits. Heating the jacket of the vessel has the advantage of increasing the heat transfer capacity which shortens the cycle time, and the heat transfer may be aided by having a flow of heating medium in the jacketed section of the vessel guided with internal baffles to ensure efficient heat transfer from the thermal fluid to the inside and contents of the vessel, as shown in the figure below. In order to optimize the cycle time, it is advantageous to optimize this indirect heat transfer.

In one embodiment of the invention, heating the contents of the autoclave is started as soon as the door is closed.

In a preferred embodiment of the invention, the total thermal energy input (steam and thermal fluid supplied by heating elements) per tonne of input municipal waste can be as low as 100 kWh, while achieving treated waste that has a low inorganic content in the biomass (or fibrous) fraction.

The average total energy input preferably is about 125 kWh per tonne or lower. The process can be advantageously implemented also with higher energy input, for example if sufficient steam is available. Generally, the energy requirement is about 200 kWh per tonne or lower

In another preferred embodiment, the amount of energy supplied through boiler (high pressure) steam is 80 % or less, relative to the total amount of energy supplied by system, more preferred the amount of fresh steam is about 75% or less, and even more preferred 60% or less relative to the average energy input. Generally, the amount of steam will be about 20% or more of the total energy required, preferable, about 40% or more, in order to achieve a short enough cycle time, and sufficient condensing steam to treat the MSW.

In a further preferred embodiment of the present invention, dust and volatiles are precluded to be discharged into the environment. This is an important feature for the acceptance of the technology, especially near populated areas, but is not so easy to achieve, since waste plants are often known to be the source of malodorous emissions.

In one embodiment, the complete plant, including all apparatus to treat the waste material is enclosed in a building that is kept at a lower pressure than atmospheric pressure, and which further comprises a bio-filter for cleaning of ventilation air before discharge to atmosphere.

In another preferred embodiment, only the part of the waste treatment plant which receives the waste is kept under the lower than atmospheric pressure, and the extracted air from that part of the building is passed to atmosphere through a bio-filter. The remainder of the plant is as far as necessary provided with local extract ventilation (LEV) hoods to preclude significant contamination of that part of the building's environment. The use of LEV hoods downstream of the autoclave has the further advantage that a safe environment for workers is achieved without the need for further protective measures. As the product material treated downstream of the autoclave is sterile, extraction of dust and any remaining volatile material is sufficient to achieve a completely safe working environment. It may be necessary to capture dust only in those areas where the waste stream is subjected to substantial movement, as the moist waste stream is not causing much dust.

The scrubbing or cleaning section for treating the suction air may comprise filters that will need to be replaced or cleaned. In a preferred embodiment, the waste-air stream is scrubbed with clean water to remove contaminants. This has as a further advantage, that the contaminations are caught by a component which thereafter is further treated in the process. More in particular, the resultant process water can be added to the waste when delivered into the vessel causing further treatment of this contaminated water. This has as further advantage that only one sort of liquid waste (contaminated process water) needs to be treated .

In case the process water appears to build-up organic waste, water can be discharged to waste-water treatment facilities, or can be further concentrated, and the organic fluid fraction can for example be used as organic feed in a sewage treatment digester plant. It is an advantage of the present invention, that less waste water is generated, so less affluent water has to be treated.

In one embodiment of the invention, the waste water is treated to remove entrained solids, ethanol, and other volatile organic compounds.

The treated waste generally will be subjected to a first classification step, in which large and small particles are separated. The smaller fraction of particles will constitute a fibrous fraction.

The so obtained fibrous fraction may be used as is. The fibrous fraction is sterilized, and contains relatively low amounts of heavy metals and a low amount of chlorine. For example, the fibrous fraction may be used in a biomass combustion, gasification or pyrolysis process, as organic feedstock for aerobic digestion (biological conversion by microorganisms), as filler for MDF panels and the like. However, in view of the fact that such fibrous fraction still has some inorganic material, it may be preferred to further improve the quality of such fibrous fraction.

It is a further preferred embodiment of the invention to provide a method allowing to obtain a fibrous fraction that has a very low amount of inorganic particles (non biomass), preferably such that the fibrous fraction conveys to the Renewable Obligation Certificate (or equivalent green energy premium) type-fuel requirements. In an alternative use, the fibrous fraction can be advantageously used in a number of soil amelioration processes like decontamination, substitution, manufacture and composting applications, as it contains relatively low amounts of heavy metal, and virtually no inorganic particles.

In such further embodiment of the invention, the process comprises a classification step in which the majority of the fibre product, contaminated with small glass shards and grit particles, is subjected to an air separation step on a vibrating screen, which causes the lighter particles to flow downwards, and the heavier particles to move upwards.

Preferably, the fibrous fraction that is to be classified is supplied to the middle section of the classification apparatus. The apparatus comprises an inclined bottom screen through which an air stream flows. The screen is vibrating, causing particles that lay on the screen to be transported upwards. The air stream causes lighter particles to be lifted slightly above the screen, and thereby, the lighter particles flow over a kind of air cushion downwards. The air velocity in such apparatus is relatively low in comparison to apparatus known as "air knives", and selected, in combination with the selection of tilt of the screen, and the strength of the vibration, to be sufficient to achieve an effective output, combined with an effective separation. It will be clear for the skilled man, that the above described method is only a preferred way of classifying the fibres. Other methods are available and can be used. Suitable other methods include sieving over flat screens, which is in particular effective in case the fibrous material is relatively dry. Depending on the waste and the amount and sizes of the grit, air knife technology may be suitable to reduce the amount of heavy particles.

With respect to non-biomass particulate material, these are mainly glass and stone, and some hard (generally filled) plastics. Hereinafter, this contamination is named inorganic material. This inorganic material is not to be confused with the ashes that will remain if the biomass is burned, as the biomass will contain salts, minerals etc.

Non-biomass particulate material (inorganic material) like glass particles may cause glass residue build-up in the combustion system, which may cause substantial maintenance problems. Further, also the inorganic material should be clean in the sense that preferably less than 3 vol%, and more preferably less than 1 vol% of the inorganic material is biomass waste in case the inorganic material is to be used as a recycled aggregate for purposes such as road-fill.

With the method of the present invention, it is possible to separate organic fibrous material and inorganic material in a reliable way, in which both fractions have very high levels of purity and high yield. Generally used test methods to determine the amount of heavy metals and the like are published by the European Committee for Standards on solid recovered fuels (CEN/TS or CEN/TR draft standards as issued in 2006).

In a preferred embodiment of the present invention, the treated waste, including the fibrous fraction are - partly - dried in the autoclave by the application of heat and preferably also vacuum. This has the advantage that screening is more efficient. Fibres with a moisture content of more than 60%, e.g. 65% which is normal for non-dried steam treated fibers, are much more difficult to separate. Further, drying under vacuum has the advantage that fibres are further subjected to break up by expanding moisture during drying and thereby the fibres lose integrity. This is also more advantageous than drying afterwards, as such a drying step will be at atmospheric pressure and less effective. Preferably, the content of the vessel is dried to have a fibrous fraction with a moisture content of about 55% or lower, and even more preferably about 50% or lower.

Because of the further degradation of the fibre, it is possible, and preferred, to remove about 10 mm or less as the smallest fraction, while still achieving high yield. It is even more preferred to remove the about 8 mm fraction or less with >90% yield of biomass (fibrous) material.

Preferably, the fibrous fraction constitutes about 90 % or more of the theoretical achievable amount of biomass fraction. It actually appears possible to achieve a yield of fibrous fraction being about 95% or more of the original biomass, such as for example about 98%.

Preferably, the amount of plastic in the fibre fraction is about 3 wt% or less, preferably 2% by weight or less, more preferably about 1 % or less (calculated relative to dry material). This is an advantage, as the use of the fibres as a biomass fuel qualifying for Renewable Obligation Certificates (or equivalent green energy premium) requires that >90% of the fuel energy content is from a renewable source. Plastic has a high calorific content, so only a very small amount is allowable in fibre to be used as a renewable biomass fuel. As explained above, it may be preferred to treat the waste at about 140 °C or lower, in order to achieve such low amount of plastic in the fibre. However, the allowable temperature will depend on the type of waste, and can be determined by the skilled man by simple experiments.

The fibrous fraction after the second classification generally will have a moisture content of about 55% or lower, preferably 45% or lower. Depending on the end-use, the moisture content may be further lowered in a circulating flash dryer, a fluidized bed dryer, band dryer or indirect dryer or the like.

Generally, the amount of cadmium is about 1 ppm (mg/kg dry matter) or lower; the amount of chromium is about 50 ppm or lower; the amount of copper is about 100 ppm or lower; the amount of lead is about 200 ppm, preferably about 100 ppm or lower, preferably about 40 ppm or lower; the amount of mercury is about 0.5 ppm, preferably about 0.2 ppm or lower; the amount of nickel is about 30 ppm or lower, preferably about 10 ppm or lower; the amount of zinc is about 300 ppm or lower and the amount of arsenic is about 1 ppm or lower.

Generally, the amount of chlorine is about 1% or lower, preferably about 0.6 wt% (on dry matter) or lower, and even more preferred about 0.2 wt% or lower.

The fibrous fraction may be pelletized, converted into briquettes for fuel and the like. It can also be used for compost and the like.

In a further preferred embodiment of the invention, the invention provides a method for providing green electricity by operating a power plant on site of a MSW treating plant according to the current invention, and providing about 50% or more of the energy requirement of the power plant by using the fibrous fraction having about 2 wt% or less of inorganic material, and having about 2 wt% or less of plastic material.

The term 'on-site' meaning that the distance between the power plant and the waste treatment plant is such, that transport of the fibrous fraction can be performed by pipe-line with air pressure, or transport belts or the like. Generally, the two plants will be within a distance of about 1 km or less, preferably 500 m or less, and more preferably 200 m or less.

In a preferred embodiment, the power plant uses the fibrous fraction for about 80% or more of its energy requirement, and even more preferred about 95% or more. As far as the power plant does not use the fibrous fraction (with Renewable Obligation Certificate (or equivalent green energy premium) status) from the waste treatment plant, it is preferred to use Renewable Obligation Certificate (or equivalent green energy premium) status fuel for the remainder of its energy requirements.

In a further preferred embodiment of the invention, the power plant and waste treatment plant are integrated, as to provide the heating requirement in the waste treatment plant by the steam of the power plant. This is particularly advantageous because of the steam needed in the early part of step (4). For this step, steam of preferably 5-25 bar (abs), more preferably 8-20 bar (abs) steam is suitable. Such steam would be anyhow available from the power plant.

In another preferred embodiment, the electricity necessary for substantially all the electricity requirements of the waste treatment plant are preferably provided by the on-site power plant.

In a preferred embodiment of the combined waste treatment and power plant, the energy provided as electricity to the grid, based on the fuel obtained from the waste treatment plant minus the energy used in the waste treatment plant (either as steam or as electricity) is about 1300 KW or more. For example, In such preferred embodiment, the energy provided by the fully renewable resources is able to deliver about 2500 KW, of which about 500 is used in the power plant, and about 500 KW is used in the waste treatment plant. In another embodiment of the invention, only a part of the energy of the power plant is provided by the waste treatment plant, for example 5 % or more, preferably 10 % or more, for example 15, 20, 30, 40 or 50%. In a further preferred embodiment, about 75% or more of the energy of the power plant is supplied by the fibrous fraction of the waste treatment plant.

### Short description of the drawings:

Fig. 1: Figure 1 shows a general outline of a waste plant using an autoclave with the addition of high pressure steam in the initial phase of the process.
Fig. 2: Figure 2 shows a general scheme for a vacuum system

### Detailed description of the drawings:

### Like numerals are used for like components in the different figures

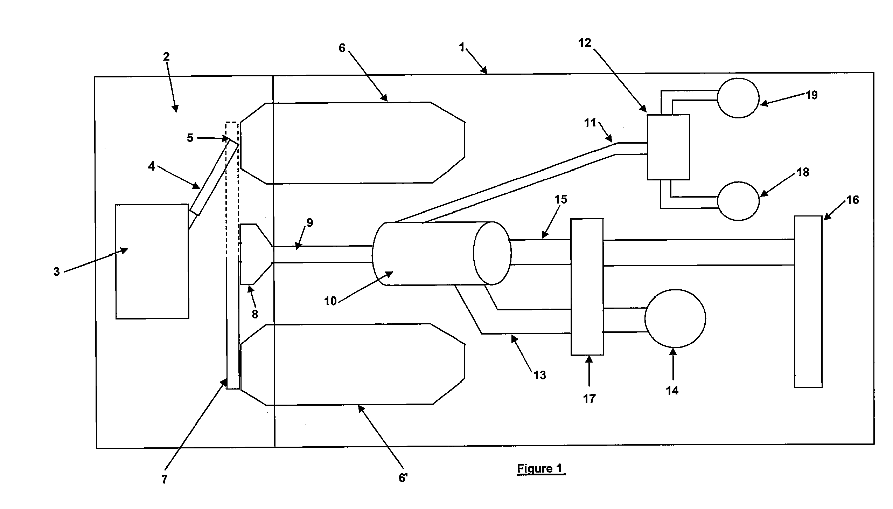

In figure 1, (1) is the plant building that -in whole or in part - may be provided with suction apparatus to maintain ventilation extract to preclude fumes to escape from the building. Air ventilation treatment may be provided through the use of bio-filters, preferably from the top of the building. Waste is provided by vehicles into area (2). In this area, a manual or automated screening is performed by an operator to take away oversized waste like engine blocks, large pieces of concrete, carpets and other large textiles or the like. Preferably, this area has active air ventilation, preferably having 2-4 air changes per hour. Further, it is preferred to have the waste fed to a hopper (3) that is provided with a screen of e.g. 40-45 cm (18 inch) mesh to further preclude oversized parts in the autoclave. Also, the hopper is used to weigh the amount of waste fed to the autoclave. The hopper may be equipped with a measurement of the weight and/or the volume to be put into the autoclave vessel. If an autoclave vessel is available to be charged, a feed conveyer (4) is used to feed the autoclave. The feed conveyer preferably is equipped with a spray nozzle (5) to have the possibility to wet the waste material. The wetting agent preferably is contaminated process water (thereby usefully recycling this water) and preferably at elevated temperature. A suitable temperature may be e.g. about 30 °C or higher, preferably about 70 °C or higher; generally, the temperature will be about 98 °C or less, preferable about 95 °C or less, and may be about 80 °C or less. The amount of water is preferably limited, and will depend on the waste to be treated. The amount may be for example about 40 wt% or less, preferably about 30 wt% or less, and even more preferably about 25 wt% or less. Generally, it will be necessary to add some water, e.g. about 5 wt% or more, or about 10 wt% or more. Because of the use of high pressure steam, it appeared that the amount of additional water can be limited. This is an advantage, because this also limits the amount of waste water effluent to be treated.

In order to improve the loading of the autoclave vessel, it may be advantageous to reduce the waste in size, e.g. by a bag-splitter or press. The size of the waste may be reduced to e.g. 15 cm or more, like 25 cm or 30 cm.

After the autoclave vessel (6) is charged, steam and optionally heat is applied to the vessel. In a preferred embodiment, the autoclave is first brought to reduced pressure, such as for example about 0.5 bar (abs) through the application of a vacuum system. Steam venting can be reduced in this way, as less air is to be replaced. Thereafter, steam is introduced, preferably from the other vessel, and the contents are vented for a short time (1-2 minutes). The vacuum system can aid in achieving fast transfer of vented gasses, and contaminated air or steam can be cleaned in a scrubber. Thereafter, the vent of the autoclave is closed.

Further steam is now introduced at high temperature, and preferably also high pressure, for example about 165 °C equivalent to about 7 bar abs or higher, preferably about 170°C, and a pressure of about 8 bar abs or higher, and even more preferably about 180 °C, and a pressure of 10 bar abs or higher. Generally, the temperature will be about 225 °C or lower and a pressure of about 25 bar or lower for practical reasons, even more preferably about 210 °C and about 20 bar or lower.

It is generally sufficient to apply the high temperature steam for only 10-20 min, like for example 15 min. Longer periods still may be acceptable, such as for example about 30 min or less. The time needed of course depends on the size of the vessel, the weight of contents and the capacity of the pipes and the steam generator.

The use of such high temperature steam allows (1) quick heat up (2) aid the formation of fibres and (3) keeps a condensing atmosphere in the vessel so that plastics do not stick to walls. It may also reduce the amount of steam required as a total to convert the organics into fibre - this in turn is useful since (4) it reduces the additional water required which (5) means less of an effluent problem and/or moisture in the residual waste and fibre.

Furthermore, in a further embodiment, indirect heating is applied as well, as it may have particular advantages in the heating and evaporation phase.

Furthermore, the content of the vessel is mixed, preferably through rotating the vessel. It would also be possible to have a mixing shaft internally in the vessel.

Rotation of the vessel generally is between 0 and 15 rpm. The vessel may be stationary during part of the process. The rotation speed during the cooking cycle may be between about 1 and 10 rpm, preferably 1.5-5 rpm. It is preferred that vessel is rotated clockwise and counterclockwise alternately during cooking. A higher speed preferably is used during unloading.

General process conditions are a temperature of about 127 °C (261 °F) or higher to achieve efficient sterilization, which temperature generally conforms with a pressure of about 2.5 bar (abs) or higher. Preferably the temperature is about 134 °C (273 °F) or higher and a pressure of about 3.0 bar (abs) or higher to achieve faster degradation of the waste material. Generally, the temperature will be about 148 °C or lower (and a pressure of about 4.8 bar (abs) or lower) as that leads to good treatment/conversion to fibre. It is particularly preferred to keep the temperature at about 145 °C (293°F) or lower, and a pressure of about 4.1 bar (abs), and even more preferred is a pressure of about 3.5 bar (abs), and a temperature of about 139 °C. This is preferred to keep the contamination of the organic fibrous fraction with plastic low. A low contamination is preferred to allow the fibrous fraction to conform with Renewable Obligation Certificate (or equivalent green energy premium) requirements. The allowable temperature will depend on the type of waste, and a higher temperature may be advantageous for shorter cooking cycles. With the teaching of the present patent disclosure, a man skilled in the art will be able to determine the maximum allowable temperature to obtain an organic fraction obeying the Renewable Obligation Certificate (or equivalent green energy premium) requirements.

The temperature and pressure are maintained at a required level by further steam injection or indirect heating. The required level preferably is a range, like for example about 3.5-4 bar (abs) and about 140-145 °C, or for example about 4-4.5 bar (abs) and about 145-148 °C. Steam injection will be performed at a substantially reduced rate in comparison with the initial steam injection during the heating and pressurization step.

It is furthermore preferred not to apply a severe milling or shredding steps, also not during any pre-treatment. This is important to preclude the setting free of heavy metals from batteries or other potentially contaminating constituents. Also, it is preferred, not to apply high temperature, as that causes plastics to melt and intermingle with organic fibre, which causes lowering of fibre yield, and contaminating the fibre and plastic fractions.

The cooking cycle may take about 15 min or more, and preferably is about 25 min or more in order to achieve a good sterilization and break-down effect. Generally, the cooking cycle (having the high pressure and temperature at a level of above 2.5 bar (abs)) will be about 2 hours or less for economic reasons.

After sufficient treatment of the waste in the autoclave, the content may be brought to atmospheric pressure and discharged on a conveyor (7), which transports the content to the down-stream classification area. It is preferred to dry the content in the autoclave through applying reduced pressure (to e.g. about 0.5 bar (abs)) and preferably by also applying indirect heating. It is furthermore preferred, to cool down the content of the vessel by allowing fresh air into the vessel, while the vacuum pump is still working. Cooling down has the advantage that VOC's, odors, or large amounts of steam are not, or less so, released into the building. Cooling down preferably is done after drying of the fibers.

It is preferred to transport the contents into an intermediate hopper (8) in order to achieve fast discharge of the autoclave vessel. The intermediate hopper (8) thereafter supplies the downstream classification area through conveyer (9). The classification device may comprise suitable picking apparatus to discharge e.g. rags, which may be added to conveyer (9). Furthermore, it may be useful to have a large size screen (e.g. 14- 30 cm (6-12 inch) screen) to remove large items before entering the classification section.

It is preferred to have as a first step in the classification area a trommel screen (10). Other screening devices may be suitable as well.

A trommel screening device (10) or other device (or plurality of devices) can have two or a plurality of classification sizes. In one embodiment of the invention, the lowest size fraction is about 12 mm or lower, although a size of about 10 mm or lower is preferred, and a size of about 8 mm or lower is even more preferred.

The fibrous fraction, obtained as lowest size fraction, can be further purified if needed. According to the figure, the fraction is transported via conveyer (11) to air classifier (12).

The next fraction (middle fraction) will be generally about 50 mm or less, and may be about 40 mm or less. This fraction may be transported via conveyer (13) to hopper (14). This will leave the oversize fraction of 4 or 5 cm or more, which can be transported over conveyer (15) to picking belt (16). On the picking belt, it is possible to remove plastic bottles, which are now clean and all labels are removed. Large wooden parts and other items may be collected. Both the large particle fraction, as the over-sized fraction, but preferably all fractions, can be subjected to magnetic and Eddy current screening devices (depicted as 17) in order to remove iron, aluminum and other metals, batteries and the like. It may be suitable, to first have certain items picked from the picking belt, before collecting metals.

The low size fraction preferably is subjected to a further classification step in order to remove grit and glass shatters in classifier (12), which will give heavies (18), that may be combined with the larger fraction in (14), and which gives fibres (19). The fibre fraction may be further treated to produce pellets or briquettes, or it can be used in loose form. Generally, one further classification will suffice, but two or three classification devices are not excluded.

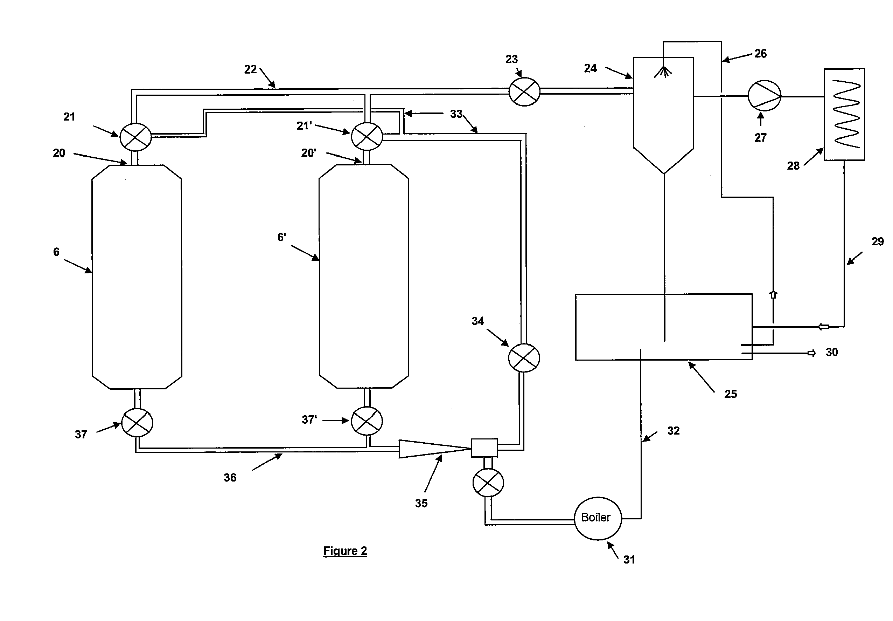

In Figure 2, vessels 6 and 6' are the autoclaves. It will be understood by the skilled man, that two or more vessels are preferred, but that the vacuum system could advantageously be used in single vessel plants. The autoclaves are equipped with vent openings (20) and (20') that are preferably located in or near the doors of the vessels. Each vent opening can be closed using manifolds (21) and (21'). The vents are connected to the vent line (22). The vent line (22) can be used to evacuate one of the vessels, e.g. vessel (6), if valve (23) and (21) are open. The contaminated air stream from the vessel is cooled and scrubbed in a scrubber (24) with water from the water tank (25) through line (26) with a pump if necessary (not shown). The vacuum pump 27 allows air to be emitted through an entrainment separator (28), the effluent water being supplied via line (29) to the water tank (25). Vent air from entrainment separator (28) passes into the LEV extraction system for the building wherein this air could be further washed using clean water, filtered or otherwise treated alongside the contaminated air streams, e.g. arising from hoods and other places.

Water in the water tank (25) can be used to pre-wet the feed to the autoclave through line (30), to feed the steam generator (31) through line (32). Boiler steam can be applied to vessel (6) after vessel (6) has been evacuated. In that case, valve or manifold (20') of vessel (6') can be opened to line (33), while opening valve (34). The supply of the steam can be aided by the use of a thermocompressor (35) or other means through line (36) to vessel (6) by opening valve (37), but this is optionally. The vent line of vessel (6) can be kept open for a few minutes to thoroughly ensure all residual air in vessel (6) is removed and a homogeneous steam atmosphere is achieved. Then, the valve (20) will be closed, and further steam is added to vessel (6) from the boiler (31), after closing valve (34). It may be preferred to have an accumulator for buffering steam capacity.

The fibrous fraction preferably has a low heavy metal content, such as for example less than 1 ppm Arsenic, and <50 ppm Lead. Typical values are 12 ppm (mg/kg dry matter) chromium, <0.5 ppm cadmium, 15 ppm lead, 0.05 ppm mercury, 35 ppm copper, 200 ppm zinc and < 1 ppm arsenic on dry matter.

## Claims

1. Process to treat municipal solid waste comprising the steps of,
(1) feeding waste material into a pressure vessel
(2) closing the vessel and optionally evacuating the contents of air
(3) agitating the vessel while having steam pressure in the vessel at about 2.5 bar (abs) or higher, and at about 4.8 bar (abs) or lower, and a temperature of about 127 °C or higher and of about 148 °C or lower for at least about 15 min
(4) reducing the pressure in the vessel
(5) optionally evacuating the contents of the vessel to 0.6 bar abs or lower
(6) discharging the vessel
(7) classifying the treated waste
wherein after step (2) steam at a temperature of about 165 °C or more and a pressure of about 5 bar (abs) or more, is injected into the vessel after closing to raise the pressure within the vessel to 2.5-4.8 bar (abs) in a condensing atmosphere, and thereafter in step (3) preferably, for a suitable period continue to inject steam at a reduced flowrate to allow heating of the waste material by steam condensation, and thereby maintain the waste treatment conditions.

2. Process according to claim 1, wherein two or more vessels are used in an alternating fashion, using steam from the discharge step (4) to apply steam to another vessel.

3. Process according to anyone of claims 1-2, wherein vacuum is applied before introducing steam in the vessel, and to dry the waste after treatment.

4. Process according to any one of claims 3, wherein a vacuum of 0.5 bar (abs) is reached in a vessel in about 15 min or less.

5. Process according to any one of claims 1-4, wherein exhaust gas is cleaned in a scrubber or a condenser.

6. Process according to any one of claims 1-5, wherein the fluid used to clean the exhaust gas is process water, which is used - in part - to wet the waste which is fed to the vessel.

7. Process according to any one of claims 1-6, wherein the waste is treated in step (3) at a temperature of about 145 °C; and at a pressure of about 4 bar abs.

8. Process according to any one of claims 1-7, wherein steam is used at a temperature of about 170 °C or higher, preferably about 180 °C or higher.

9. Process according to any one of claims 1-8, wherein high temperature steam is introduced for less than 30 min.

10. Process to treat municipal solid waste according to any one of claims 1 - 9 in two or more vessels, which are used in an alternating fashion, comprising the steps of, depressurising the vessel, and transferring the steam from a first vessel to a second vessel with the aid of a compressor

11. Process to treat municipal solid waste, according to any one of claims 1 - 10 further comprising the steps of, heating the content of the vessel through indirect heating with thermal fluid.

12. Process according to any one of claims 11, wherein indirect heating is applied through conduits in the vessel, and through a heated jacket of the vessel.

13. Process according to any one of the claims 11-12, wherein during discharge of the vessel, the inner surface and the coils of the vessel are at a temperature of about 110 °C or below.

14. Integrated power plant and waste treatment plant, wherein the waste treatment plant is a plant able to perform a process according to any one of claims 1-13, and wherein the heating requirement in the waste treatment plant is provided by the steam supply of the power plant.

15. Method for providing green electricity by operating a power plant on site of a MSW treating plant able to perform a process according to any one of claims 1-13, and providing about 5% or more of the energy requirement of the power plant by using the fibrous fraction having about 2 wt% or less of inorganic material, and having about 2 wt% or less of plastic material.
